# EUROPÄISCHE PATENTSCHRIFT

(11) **EP 4 069 320 B1**
(45) Veröffentlichungstag und Bekanntmachung des Hinweises auf die Patenterteilung: **02.04.2025**
(21) Anmeldenummer: 20804248.1
(22) Anmeldetag: 10.11.2020
(51) Int. Cl.: A61L 15/22, A61F 13/00, A61K 8/02, A61B 90/80, D04H 1/70

(54) **ARTIKEL ZUR MECHANISCHEN WUNDREINIGUNG**
ARTICLE FOR MECHANICAL DEBRIDEMENT
ARTICLE POUR DÉBRIDEMENT MÉCANIQUE

(30) Priorität: 05.12.2019 DE 102019133239
(43) Veröffentlichungstag der Anmeldung: 12.10.2022
(73) Patentinhaber: Carl Freudenberg KG, 69469 Weinheim (DE)
(72) Erfinder: LANG, Birthe, 69469 Weinheim (DE); SCHLESSELMANN, Bernd, 69469 Weinheim (DE); ELLMER, Katharina, 20900 Monza (IT)
(86) Internationale Anmeldenummer: PCT/EP2020/081583
(87) Internationale Veröffentlichungsnummer: WO 2021/110371

(56) Entgegenhaltungen:
- EP-A1- 3 133 196
- WO-A1-2013/113906

## Beschreibung

Die Erfindung betrifft einen sterilen Artikel zur mechanischen Wundreinigung, ein Verfahren zu seiner Herstellung, sowie seine Verwendung als Reinigungsmittel insbesondere für eine menschliche oder tierische Körperoberfläche.

In der Wundbehandlung spielt die Wundreinigung (Debridement), das heißt die Reinigung der Wunde von nekrotischem (avitalem), infiziertem oder geschädigtem Gewebe, Fibrinbelägen, Wundexsudat und sonstigen Verschmutzungen eine integrale Rolle. Nekrotisches Gewebe und/oder eine starke Präsenz von Bakterien, können den Heilungsprozess einer Wunde stark behindern. Daher ist es medizinisch angeraten ein Debridement, insbesondere bei chronischen Wunden, zwischen jedem Verbandswechsel durchzuführen. Es gibt verschiedene Methoden, die zur Wundreinigung eingesetzt werden: autolytisch, mechanisch, chirurgisch, biochirurgisch. Bei allen Methoden ist es essentiell, dass bei der Entfernung des abgestorbenen Gewebes bereits neu entstandenes Granulations- und junges Epithelgewebe, welche sich bei Wundschluss bilden, nicht beschädigt werden.

Eine Methode, die zunehmend verwendet wird, ist die mechanische Wundreinigung. Hierbei werden verschiedene Flächengebilde, zum Beispiel Textilien oder Schwämme, zur Reinigung der Wunde eingesetzt. Ein Vorteil der mechanischen Wundreinigung ist, dass sie einfach durchzuführen ist und auch in der Heimpflege von Pflegekräften angewendet werden kann.

Die Anforderungen an Flächengebilde für die mechanische Wundreinigung sind vielfältig. Zum einen müssen sie einen auf die Anwendung abgestimmten abrasiven Charakter haben, der ein sanftes schmerzfreies Entfernen nekrotischen Gewebes und von Belägen ermöglicht, zum anderen ein gutes Absorptionsvermögen aufweisen, das eine schnelle Aufnahme der gelösten Wundbeläge ermöglicht und so eine erneute Kontamination der Wunde verhindert. Des Weiteren sollte die Struktur flexibel sein, so dass ein Reinigen verschiedenster Wundgeometrien möglich ist, d.h. sowohl großflächiger Wunden als auch von Tunnelgängen.

Die Verwendung verschiedener Flächengebilde zur mechanischen Wundreinigung ist bereits bekannt.

Ein kommerziell erhältlicher Schaum zum mechanischen Debridement ist der Wundputzer^{®} von Ligasano^{®}. Der retikulierte hydrophobe Schaum zeigt eine gute Reinigungsleistung aufgrund seiner stark abrasiven Struktur. Diese Struktur kann aber auch zu starken Schmerzen beim Patienten während der Anwendung führen. Der retikulierte Schaum weist eine hohe Offenporigkeit auf, um feste und hoch viskose Wundbestandteile zu entfernen. Dies führt jedoch zu einem verringerten Absorptionsvermögen.

Neben Schäumen ist auch die Verwendung von textilen Flächengebilden zur Wundreinigung beschrieben.

Die EP 2 365 794 B1 beschreibt eine Wundreinigungseinrichtung, welche ein Tuch aufweist, oder ist, welche eine Trägerschicht mit auskragenden oder daran angeordneten Fäden aus synthetischen Fasern aufweist, die vorzugsweise abgeschnittene Enden bzw. Endflächen aufweisen. Nachteilig ist, dass die Reinigungswirkung durch die auskragenden Fasern erzielt wird. Dies führt schnell zu einem Verkleben der Faserenden, insbesondere bei Wunden mit viskosem Exsudat, was eine schnelle Erschöpfung der Kapazität des Tuches zur Folge hat. Dazu kommt, dass die zwischen den Fasern bereitgestellten Hohlräume, aufgrund ihrer länglichen Geometrie nicht so gut zur Aufnahme und Einschluss der Wundbestandteile geeignet sind.

Ein kommerziell verfügbares Produkt des vorgenannten Aufbaus, ist das Wundreinigungs-Produkt Debrisoft^{®}. Zusätzlich zu den obengenannten Nachteilen ist nachteilig, dass das Produkt harte, umsäumte Kanten aufweist, die beim Kontakt mit dem Wundgrund starke Schmerzen verursachen können. Die Kanten sind jedoch notwendig zum Zusammenhalten der textilen Struktur, da beim Zuschneiden des Produktes Fasern freigesetzt werden, die zu einer Kontamination der Wunde führen können. Dazu kommt, dass es eine hohe Steifigkeit hat, so dass es sich nur für den Einsatz auf flächigen Wunden eignet. Ferner wird Exsudat nur an der Oberfläche gebunden und nicht in der Struktur des Tuches aufgenommen. Schließlich ist es nur einseitig benutzbar.

US 20030079324 A1 beschreibt ein Verfahren zur Herstellung eines mittels Wasserstrahl verfestigten strukturierten durchlässigen Vliesstoffs mit hohem Absorptionsvermögen und guter Abrasion insbesondere im feuchten Zustand. Nachteilig ist hier die durch den Herstellungsprozess bedingte geringe Dicke des Materials, wodurch nur wenig Raum zur Aufnahme und zum Einschluss der Wundbestandteile zur Verfügung steht.

Ein kommerziell verfügbares und weit verbreitetes mechanisches Wundreingungs-Produkt auf Faserbasis ist Baumwollgaze. Diese ist ein offenes Gewebe aus Baumwollgarnen und zeichnet sich zum einen durch ihren günstigen Preis und zum anderen durch eine hohe Versatilität aus. Nachteilig an diesem Produkt ist, dass es aufgrund der groben Garnstruktur in der Anwendung schmerzhaft für den Patienten sein kann. Darüber hinaus besteht die Gefahr einer Verunreinigung der Wunde durch sich aus dem Garn lösende Faserbestandteile, insbesondere beim Zuschneiden der Gaze.

Die WO 2013/113906 A1 beschreibt einen Wundpflegeartikel, aufweisend mindestens eine Oberfläche mit abrasiven Eigenschaften, die so ausgebildet ist, dass der Wundpflegeartikel geeignet ist, bei Relativbewegung desselben zu einer Wunde in der Wunde angeordnete Biofilme aufzubrechen und/oder die Wundexsudation anzuregen. Die Oberfläche mit abrasiven Eigenschaften kann ein Schaumstoff oder ein Textil sein. Ebenfalls beschrieben ist ein Produkt, das sowohl einen Polyurethanschaum als auch auf diesen kaschiertes textiles Material enthält. Nachteilung an diesem Verbundmaterial ist seine hohe Steifigkeit und Delaminierungsneigung. Darüber hinaus wird durch die Kaschierung ein Transport von Wundexsudat durch die Struktur erschwert.

Ein kommerziell erhältliches Produkt des vorgenannten Aufbaus ist das Wundreinigungs-Produkt Cutimed^{®} DebriClean. Zusätzlich zu den im vorangehenden Absatz genannten Nachteilen weist das Produkt den Nachteil auf, dass es harte Kanten sowie eine hohe Steifigkeit aufweist, wodurch es sich nur für großflächige Wunden eignet. Ferner ist es nur einseitig benutzbar.

Der Erfindung liegt die Aufgabe zu Grunde, einen Wundreinigungsartikel bereitzustellen, mit dem die vorgenannten Nachteile zumindest teilweise ausgeräumt werden können. Insbesondere soll der Wundreinigungsartikel eine gute und schonende Wundreinigung ermöglichen. Dabei sollen nekrotisches Gewebe und Beläge ohne starke Krafteinwirkung entfernt werden können, so dass die Gefahr junges Granulationsgewebe zu verletzten minimiert wird. Darüber hinaus soll der Wundreinigungsartikel zu entfernende Wundbestandteile sicher in der Struktur aufnehmen und einschließen, so dass eine Rekontamination der Wunde während des Reinigungsvorgangs verhindert wird. Außerdem soll der Artikel für verschiedene Wundgeometrien, beispielsweise großflächige oder tunnelförmige Wunden, einsetzbar sein. Ferner soll der Artikel zuschneidbar und ohne signifikanten Verlust von Fasern einsetzbar sein.

Diese Aufgabe wird gelöst durch einen sterilen Artikel zur mechanischen Wundreinigung, umfassend einen Vertikalvliesstoff mit einer Dicke von 1,5 mm bis 50 mm, wobei der Vertikalvliesstoff Bindefasern enthält, wobei mindestens eine Seite des Vertikalvliesstoffs für den direkten Kontakt mit der Wunde vorgesehen ist, wobei der Vertikalvliesstoff vertikal zur Richtung seiner Dicke gefaltet ist.

Unter mechanischer Wundreinigung ist die Reinigung einer Wunde mittels mechanischer Einwirkung, d.h. repetitiver Bewegung eines Reinigungsartikels unter leichter Druckeinwirkung (bspw. Auswischen, Reiben) zu verstehen.

Unter einem Vertikalvliesstoff wird erfindungsgemäß ein Vliesstoff verstanden, der eine vertikale Komponente enthält, die aus dessen spezifischem Herstellungsprozess resultiert. Unter einem Vliesstoff wird dabei ein Vliesstoff gemäß DIN EN ISO 9092:2012-01 verstanden. Im Gegensatz zu einem klassischen Vernadelungsverfahren, bei dem in der Regel nur eine lokale vertikale Ausrichtung eines horizontalen Faserflors in den Einstichkanälen der Nadeln erzielt wird, wird bei der Herstellung eines Vertikalvliesstoffs in der Regel der gesamte gebildete Faserflor in vertikaler Richtung gefaltet und so vertikal zur Faserflorebene ausgerichtet. Erfindungsgemäß ist der Vertikalvliesstoff daher gefaltet. Er ist im Wesentlichen vertikal zu seiner Grundfläche gefaltet, d.h. vertikal zu derjenigen Fläche des Vertikalvliesstoff gefaltet, die eine Abmessung von 1,5 mm bis 50 mm aufweist. Gefaltet bedeutet dabei mindestens eine und vorzugsweise mehr als eine Faltung, insbesondere zahlreiche Faltungen. Vorzugsweise findet die Faltung ziehharmonikaartig statt.

Die Fasern beziehungsweise der Flor müssen dabei nicht einer einzigen Geraden beziehungsweise Ebene folgen, sondern können beispielsweise gebogen sein oder einem Zick Zack Verlauf folgen. Verglichen mit nach herkömmlichen Verfahren hergestellten Vliesstoffen, weisen Vertikalvliesstoffe eine voluminösere Porenstruktur auf. Beispielhafte, erfindungsgemäß geeignete Herstellungsverfahren für Vertikalvliesstoffe sind in den Dokumenten US 2016/0244895 A1 (V-Lap Verfahren), US 8,357,256 B2 (Wavemaker Santex Verfahren) und CN 104805597 (Anyou-Verfahren) zu finden.

Erfindungsgemäß wurde gefunden, dass sich ein Vertikalvliesstoff hervorragend für die mechanische Wundreinigung eignet, da die vertikal orientierten Fasern eine schnelle Aufnahme von Wundexsudat und somit eine effiziente Reinigung ermöglichen. Zusätzlich erlaubt die voluminöse Porenstruktur im Vertikalvliesstoff einen sicheren Einschluss von Wundbestandteilen. Darüber hinaus können die vertikal ausgerichteten Fasern besonders gut während der Reinigung auftretende Druckbelastungen aufnehmen und verteilen, da die vertikal ausgerichteten Fasern einer Komprimierung des Vertikalvliesstoffs entgegenwirken. Hierdurch kann eine für den Patienten schonendere Reinigung ermöglicht werden, da auftretende Belastungsspitzen auf der Wundoberfläche reduziert werden. Ferner erlaubt die durch die Faltung bedingte inhärente Elastizität eine gute Anpassung des Wundmaterials an den Wundgrund, wodurch eine sehr gute Reinigungswirkung erzielt werden kann.

Erfindungsgemäß ist mindestens eine Seite des Vertikalvliesstoffs zum direkten Kontakt mit der Wunde vorgesehen. Aus diesem Grund weist diese Seite vorzugsweise zumindest beim Einsatz in der Wundreinigung keine weitere Schicht auf, die den direkten Kontakt zur Wunde verhindern würde. In einer bevorzugten Ausführungsform der Erfindung sind beide Seiten des Vertikalvliesstoffs zum direkten Kontakt mit der Wunde vorgesehen und weisen deshalb vorzugsweise zumindest beim Einsatz in der Wundreinigung keine weitere Schicht auf. Durch den direkten Kontakt des Vertikalvliesstoffs mit der Wunde können dessen vorteilhafte Eigenschaften für die Wundreinigung besonders gut zur Wirkung kommen. Mithin weist vorzugsweise mindestens eine, insbesondere beide Seiten des Vertikalvliesstoffs keine mit dem Vertikalvliesstoff unlösbar verbundene Schichten auf. Unter unlösbar ist erfindungsgemäß zu verstehen, dass die Verbindung der Schichten so stark ist, dass sie nicht einfach händisch, bspw. vom Pflegepersonal, gelöst werden kann.

Dennoch ist es denkbar, dass der Vertikalvliesstoff auf einer oder beiden Seiten beispielsweise für Transport oder Verpackungszwecke weitere Schichten aufweist. Um einen direkten Wundkontakt zu ermöglichen, sollte dann aber zumindest eine Schicht lösbar mit dem Vertikalvliesstoff verbunden vorliegen. Unter lösbar ist erfindungsgemäß zu verstehen, dass die Schicht einfach händisch gelöst werden kann. Beispiele für lösbare Schichten sind schwachklebende Schutzfolien. Diese Schicht kann dann vor dem Kontakt mit der Wunde entfernt werden.

Der Vertikalvliesstoff kann die verschiedensten Matrixfasern enthalten. Unter Matrixfasern sind erfindungsgemäß Fasern zu verstehen, die im Vertikalvliesstoff nicht, oder nur zu einem geringen Teil thermisch verschmolzen vorliegen. Bevorzugt sind die Matrixfasern thermoplastische Matrixfasern, die vorzugsweise einen Schmelzpunkt der am niedrigsten schmelzenden Faserkomponente von über 100°C, beispielsweise von 100°C bis 200°C, noch bevorzugter von über 200°C aufweisen. In einer Ausführungsform bestehen die Matrixfasern aus einer Faserkomponente. In einer weiteren bevorzugten Ausführungsform bestehen die Matrixfasern aus mehreren Faserkomponenten. Besonders bevorzugt sind die Matrixfasern Vollprofilfasern, Multilobalvollprofilfasern, Hohlfasern, Vollprofilbikomponentenfasern (z.B. Kern/Mantel, Side-by-Side). Vorteilhaft an der Verwendung von Multilobalvollprofilfasern beispielsweise Trilobalfasern ist, dass über die Fasergeometrie die Aufnahme und der Transport von Wundbestandteilen besonders gut ermöglicht werden kann.

Geeignete Polymerklassen zur Herstellung der Matrixfasern können unter anderem Polyester, Polyamide, Polyolefine, Polyacrylnitril, Cellulose und/oder Polyvinylalkohole sein. Bevorzugt sind Polyester, Polyamide, Polyolefine, Polyacrylnitril, und/oder Polyvinylalkohole.

Der Anteil der Matrixfasern im Vertikalvliesstoff beträgt vorzugsweise mindestens 20 Gew.%, beispielsweise von 20 Gew.% bis 100 Gew.%, noch bevorzugter mindestens 25 Gew.%, beispielsweise von 25 Gew.% bis 80 Gew.%, noch bevorzugter mindestens 30 Gew.%, beispielsweise von 30 Gew.% bis 70 Gew.%, jeweils bezogen auf das Gesamtgewicht des Vertikalvliesstoffs.

Erfindungsgemäß weist der Vertikalvliesstoff Bindefasern auf. Bindefasern sind Fasern, die im Vertikalvliesstoff zumindest teilweise thermisch verschmelzbar und/oder verschmolzen sind und hierdurch Bindepunkte zwischen den Fasern schaffen. Vorteilhaft an der Verwendung von Bindefasern ist, dass diese die 3-dimensionale Struktur des Vertikalvliesstoffes fixieren können, ohne dass eine Veränderung der Faserorientierung oder des Porenvolumes stattfindet, wie es beispielsweise beim mechanischen Vernadeln vorkommt. Vorzugsweise ist der Vertikalvliesstoff thermisch verfestigt.

Erfindungsgemäß bevorzugt weist der Vertikalvliesstoff zumindest teilweise thermisch verschmolzene Bindefasern auf. Vorzugsweise weist mindestens eine schmelzbare und/oder verschmolzene Faserkomponente der Bindefaser, insbesondere eine außen angeordnete schmelzbare und/oder verschmolzene Faserkomponente, einen Schmelzpunkt auf, der niedriger liegt als der Schmelzpunkt anderer im Vliesstoff enthaltener Faserkomponenten, insbesondere niedriger als der Schmelzpunkt der am niedrigsten schmelzenden Faserkomponente der Matrixfasern.

Weist die Bindefaser mehrere schmelzbare und/oder verschmolzene Faserkomponenten auf, so liegt der Schmelzpunkt der am höchsten schmelzenden Faserkomponente der Bindefaser bevorzugt mehr als 10°C, besonders bevorzugt mehr als 30°C unter dem Schmelzpunkt der am niedrigsten schmelzenden Faserkomponente der Matrixfasern. Geeignete Bindefasern weisen einen Schmelzpunkt der am höchsten schmelzenden Faserkomponente von unter 250°C, beispielsweise von 100°C bis 200°C, noch bevorzugter von unter 180°C, beispielsweise von 100°C bis 180°C, insbesondere unter 175°C, beispielsweise von 100°C bis 175°C auf.

Sofern der Vertikalvliesstoff Bindefasern in Kombination mit Matrixfasern aufweist, ist es vorteilhaft, wenn der Schmelzpunkt der in der Matrixfaser enthaltenen Faserkomponente mit dem niedrigsten Schmelzpunkt über dem Schmelzpunkt der in der Bindefaser enthaltenen Faserkomponente mit dem höchsten Schmelzpunkt liegt. Vorzugsweise liegt der Schmelzpunkt der Faserkomponente mit dem niedrigsten Schmelzpunkt in der Matrixfaser mindestens 10°C, besonders bevorzugt mindestens 30°C, über dem Schmelzpunkt der Faserkomponente mit dem höchsten Schmelzpunkt in der Bindefaser.

Bevorzugte schmelzbare und/oder verschmolzene Faserkomponenten in Bindefasern sind Polyolefin, Polyester, Polyamid und/oder Gemische hiervon sowie Copolymere wie Ethylen-Vinylacetat-Copolymere. Ebenfalls bevorzugte Bindefasern sind Bikomponentenfasern, insbesondere Bikomponentenfasern, die Polyolefin, Polyester, Ethylen-Vinylacetat-Copolymere, Polybutylenterephtalat, und/oder Gemische hiervon als außen angeordnete Faserkomponente enthalten. Die Bindefasern können unterschiedliche Querschnittsgeometrien wie Vollprofilfaser-, Multilobalvollprofilfaser-, Hohlfaser-, Vollprofilbikomponentenfaser- (z.B. Kern/Mantel, Side-by-Side) geometrien aufweisen. Bevorzugt sind Schmelzbindefasern in Form von Vollprofilfasern. Vorteilhaft an Bikomponentenfasern ist, dass nur ein Teil der Faser aufschmilzt und dadurch eine weniger biegesteife und flexiblere Struktur erhalten wird.

Der Anteil der Bindefasern im Vertikalvliesstoff beträgt bevorzugt mindestens 20 Gew.%, beispielsweise von 20 Gew.% bis 100 Gew.%, noch bevorzugter mindestens 25 Gew.%, beispielsweise von 25 Gew.% bis 80 Gew.%, noch bevorzugter mindestens 30 Gew.%, beispielsweise von 30 Gew.% bis 70 Gew.%, jeweils bezogen auf das Gesamtgewicht des Vertikalvliesstoffs.

**In** einer bevorzugten Ausführungsform der Erfindung weist der Vertikalvliesstoff gekräuselte Fasern auf. Vorteilhaft hieran ist, dass gekräuselte Fasern einer Druckverformung des Vertikalvliesstoffes entgegenwirken, was die Stabilität der voluminösen Porenstruktur gegenüber einer Druckbelastung und die Druckverteilungskapazität weiter erhöht.

Geeignete gekräuselte Fasern sind 2-dimensional gekräuselte Fasern wie beispielsweise die "Grisuten"-Polyesterfasern der Firma Märkische Faser GmbH in Premnitz. Besonders geeignet sind 3-dimensional gekräuselte Fasern, (z.B. spiral gekräuselte Fasern), weil sie gegenüber der 2-dimensional gekräuselten Faser ein erhöhtes Rückerholungsvermögen aufweisen. Die 3-dimensionale Kräuselung kann schon beim Spinnprozess erzeugt werden, beispielsweise wie die "Softflex HY" von Indorama, oder entsteht bei thermischer Beaufschlagung einer Bikomponentenfaser beispielsweise mit einer side-by-side oder exzentrischen Kern-Mantel Querschnittsgeometrie, beispielsweise wie die "EMF" Faser von Huvis.

Der Anteil der gekräuselten Fasern im Vertikalvliesstoff beträgt bevorzugt mindestens 20 Gew.%, beispielsweise von 20 Gew.% bis 100 Gew.%, noch bevorzugter mindestens 25 Gew.%, beispielsweise von 25 Gew.% bis 80 Gew.%, noch bevorzugter mindestens 30 Gew.%, beispielsweise von 30 Gew.% bis 70 Gew.%, jeweils bezogen auf das Gesamtgewicht des Vertikalvliesstoffs.

In einer bevorzugten Ausführungsform der Erfindung weisen die gekräuselten Fasern eine Kräuselbogenanzahl von 4 bis 25 Bögen/cm auf, bevorzugt zwischen 6 und 18 Bögen/cm, besonders bevorzugt zwischen 8 und 14 Bögen/1 cm.

Vorteilhafterweise weist der Vertikalvliesstoff gekräuselte Bindefasern auf, insbesondere zumindest teilweise verschmolzene Bindefasern auf. Hieran ist vorteilhaft, dass gekräuselte Bindefasern besonders elastische formstabile Strukturen ausbilden. Der Grund hierfür liegt darin, dass die durch die gekräuselten Fasern vermittelte Sprungelastizität durch die ausgebildeten Bindungspunkte fixiert wird. Der Anteil der gekräuselten Bindefasern liegt vorteilhafterweise über 20 Gew.%, beispielsweise bei 20 Gew.% bis 100 Gew.%, noch bevorzugter von 30 Gew.% bis 80 Gew.% und insbesondere 30 Gew.% bis 70 Gew.%, jeweils bezogen auf das Gewicht des Vertikalvliesstoffes.

Die gekräuselten Fasern können auch Matrixfasern enthalten.

Der Vertikalvliesstoff kann auch nicht gekräuselte Fasern enthalten, beispielsweise nicht gekräuselte Fasern, die Polyester, Polyolefin, Polyamid und/oder Gemische hiervon enthalten. Bevorzugt liegen die weiteren Fasern als Stapelfasern vor.

Bevorzugt enthält der Vertikalvliesstoff Stapelfasern, vorzugsweise mit einer Stapellänge zwischen 20 mm und 150 mm, noch bevorzugter zwischen 30 mm und 90 mm und insbesondere zwischen 40 mm und 70 mm. Der Anteil der Stapelfasern liegt vorzugsweise über 60 Gew.%, noch bevorzugter über 70 Gew.%, noch bevorzugter über 80 Gew.%, insbesondere über 90 Gew.% jeweils bezogen auf das Gesamtgewicht des Vertikalvliesstoffs. Dabei können eine oder mehrere Fasertypen, bspw. Bindefasern, Matrixfasern und/oder weitere Fasern, als Stapelfasern ausgebildet sein. Vorzugsweise sind sämtliche im Vertikalvliesstoff enthaltene Fasern Stapelfasern.

Vorzugsweise weist der Vertikalvliesstoff Fasern mit einem Fasertiter im Bereich von 0,9 dtex bis 100 dtex (g/10.000 m), noch bevorzugter zwischen 1,5 dtex und 30 dtex, insbesondere zwischen 3 dtex und 11 dtex auf. Dabei können eine oder mehrere Fasertypen, bspw. Bindefasern, Matrixfasern und/oder weitere Fasern den vorgenannten Fasertiter aufweisen. Besonders bevorzugt weisen sämtliche im Vertikalvliesstoff enthaltene Fasern den vorgenannten Fasertiter auf.

In einer weiteren bevorzugten Ausführungsform der Erfindung ist der Vertikalvliesstoff thermisch verfestigt. Vorteilhaft hieran ist, dass durch die thermische Verfestigung die 3-dimensionale Struktur des Vertikalvliesstoffs fixiert werden kann, ohne die Porenstruktur oder das Volumen des Vertikalvliesstoffs zu beeinträchtigen.

Die Dicke des Vertikalvliesstoffes beträgt erfindungsgemäß 1,5 mm bis 50 mm, vorzugsweise 2 mm bis 40 mm, noch bevorzugter 2 mm bis 30 mm und insbesondere 2 mm bis 25 mm, gemessen nach DIN EN ISO 9073-2:1997-02. Das Flächengewicht des Vertikalvliesstoffes beträgt vorzugsweise 30 g/m² bis 500 g/m², noch bevorzugter 40 g/m² bis 400 g/m², noch bevorzugter 40 g/m² bis 300 g/m², noch bevorzugter 100 g/m² bis 300 g/m² und insbesondere 2 mm bis 25 mm, gemessen nach DIN EN 29073-1:1992-08.

In einer weiteren bevorzugten Ausführungsform weist der Vertikalvliesstoff eine Wundspüllösung, vorzugsweise eine sterile Wundspüllösung auf.

Ein weiterer Gegenstand der Erfindung ist ein steriler Artikel, welcher der Vertikalvliesstoff an sich ist, wobei der Vertikalvliesstoff steril ist oder steril verpackt ist. Wenn der Vertikalvliesstoff steril verpackt ist, befindet er sich in einer Umverpackung. Die Umverpackung ist bevorzugt undurchlässig für Mikroorganismen, sodass die Sterilität des Vertikalvliesstoffs auch bei Lagerung beibehalten werden kann.

Im Sinne der vorliegenden Erfindung meint "steril" insbesondere steril im Sinne von DIN 13151:2008-12.

Ebenfalls offenbart, aber nicht Teil der vorliegenden Erfindung ist ein Verfahren umfassend folgende Schritte:
1. In Kontakt bringen eines sterilen Artikels, umfassend einen Vertikalvliesstoff mit der menschlichen oder tierischen Körperoberfläche;
2. Repetitives Bewegen des Artikels auf der Körperoberfläche.

Das Verfahren eignet sich aufgrund der vertikal ausgerichteten Fasern und der voluminösen Porenstruktur des Vertikalvliesstoffs, wie oben erläutert hervorragend zur mechanischen Wundreinigung. Dabei kann mit dem repetitiven Bewegen eine besonders gründliche Reinigung erzielt werden. Vorzugsweise wird in dem Verfahren ein Vertikalvliesstoff gemäß einer oder mehrerer der hier beschriebenen Ausführungsformen verwendet.

In einer Ausführungsform ist das Verfahren nicht-therapeutisch und/oder kosmetisch. Bei dem Verfahren kommt dabei insbesondere kein Stoff oder Stoffgemisch zum Einsatz, der bzw. das eine pharmakologische, insbesondere therapeutische, Wirkung hat. Insbesondere erfolgt keine präventive, kurative und/oder palliative Behandlung eines pathologischen Zustands. Das nichttherapeutische und/oder kosmetische Verfahren kann ein Behandlungsverfahren, d.h. ein Verfahren zur Behandlung der Körperoberfläche, sein, welches seinem Charakter nach nicht therapeutisch ist. Das Verfahren ist insbesondere ein Verfahren zum Reinigen der Körperoberfläche, bevorzugt zum Reinigen einer menschlichen Körperoberfläche.

Ebenfalls offenbart, aber nicht Teil der vorliegenden Erfindung ist ein Verfahren zur mechanischen Wundreinigung umfassend folgende Schritte:
1. In Kontakt bringen eines sterilen Artikels zur mechanischen Wundreinigung, umfassend einen Vertikalvliesstoff mit einer Wunde;
2. Repetitives Bewegen des Artikels auf der Wunde.

In einer Ausführungsform besteht das jeweilige Verfahren aus den Schritten 1 und 2.

Ebenfalls offenbart, aber nicht spezifisch beansprucht ist die Verwendung eines sterilen Artikels umfassend einen Vertikalvliesstoff zur mechanischen Wundreinigung.

Ein weiterer Gegenstand der Erfindung ist eine Verwendung eines erfindungsgemäßen sterilen Artikels als Reinigungsmittel, insbesondere für eine menschliche oder tierische Körperoberfläche. Bevorzugt ist das Reinigungsmittel so ausgestaltet, dass es Verunreinigungen über seine gesamte Dicke aufnehmen kann. Bevorzugt handelt sich bei aufzunehmenden Verunreinigungen um organische, insbesondere bioorganische Verunreinigungen. Bioorganische Verunreinigungen sind von einem lebenden Organismus abgegebene Verunreinigungen.

### Figurenbeschreibung

### Kurzbeschreibung der Zeichnung

In den Zeichnungen zeigen:
Fig. 1 den Querschnitt einer Rasterelektronenmikroskopie-Aufnahme eines erfindungsgemäßen Verbundmaterials,
Fig. 2 den Querschnitt einer Rasterelektronenmikroskopie-Aufnahme eines erfindungsgemäßen Wundreinigungsmaterials nach einer Wundreinigung,
Fig. 3 den Querschnitt einer Rasterelektronenmikroskopie-Aufnahme einer Baumwollgaze nach einer Wundreinigung,
Fig. 4 den Querschnitt einer Rasterelektronenmikroskopie-Aufnahme des Produktes Debrisoft ^{®} nach einer Wundreinigung,
Fig. 5 die definierte Bewegung eines Wischzyklus zur Reinigung einer Testwunde auf Schweinehaut.

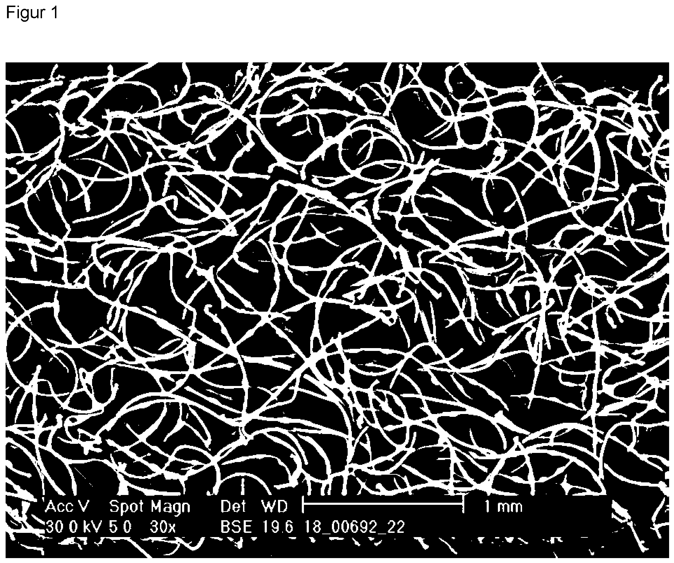

Fig. 1 zeigt den Querschnitt einer Rasterelektronenmikroskopie-Aufnahme eines erfindungsgemäßen Vertikalvliesstoffs. In der Figur ist die offenporige Struktur des Vertikalvliesstoffs deutlich zu erkennen.

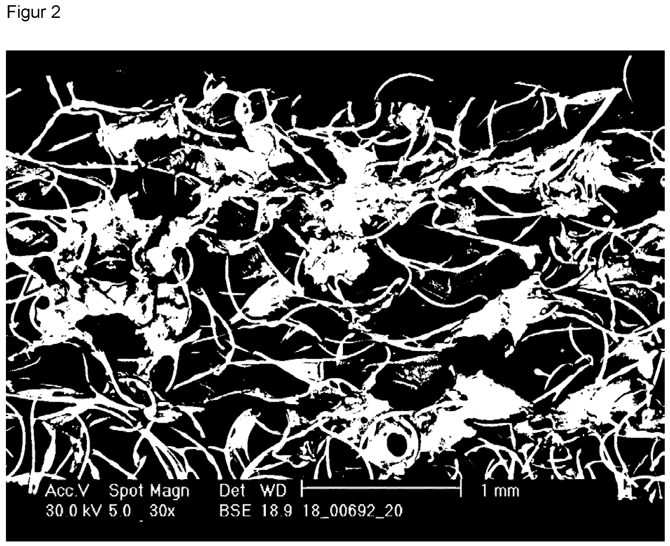

Fig. 2 zeigt den Querschnitt einer Rasterelektronenmikroskopie-Aufnahme eines erfindungsgemäßen Wundreinigungsmaterials nach einer Wundreinigung. Es ist deutlich zu erkennen, wie Wundexsudat in der Porenstruktur des Vertikalvliesstoffes eingeschlossen wird (Bereich innerhalb gepunkteter Linie).

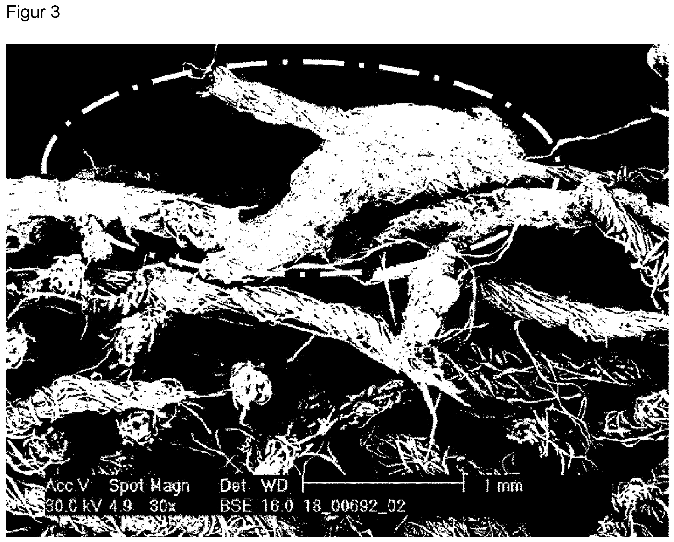

Fig. 3 zeigt den Querschnitt einer Rasterelektronenmikroskopie-Aufnahme einer Baumwollgaze nach einer Wundreinigung. Man erkennt deutlich, wie die Wundbestandteile lediglich auf der Oberfläche der Baumwollgaze festgehalten werden (Bereich innerhalb Punkt-Strich Linie).

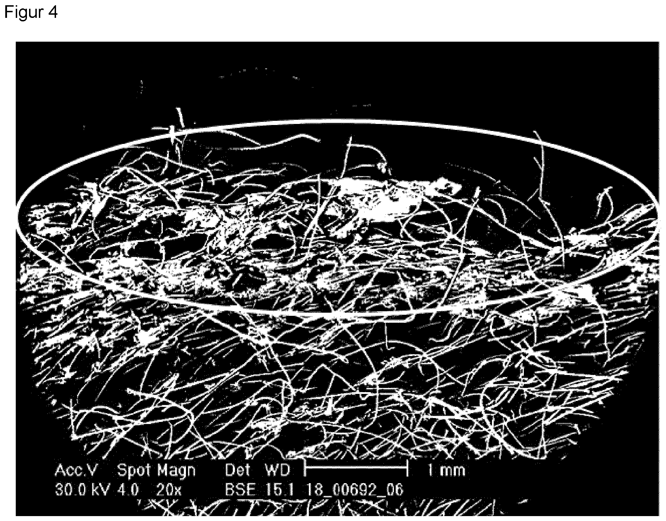

Fig. 4 zeigt den Querschnitt einer Rasterelektronenmikroskopie-Aufnahme des Produktes Debrisoft ^{®} nach einer Wundreinigung. Man erkennt deutlich, wie die Wundbestandteile lediglich auf der Oberfläche festgehalten werden (Bereich innerhalb Ovals).

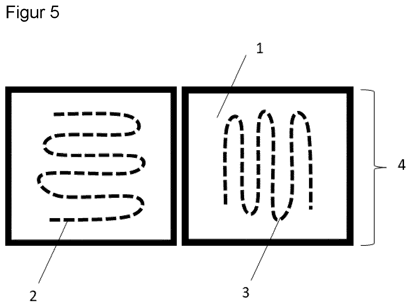

Fig. 5 zeigt die definierte Bewegung eines Wischzyklus zur Reinigung einer Testwunde auf Schweinehaut. Die Reinigung der Wundfläche 1 erfolgt mit geringem Druck (zur Simulation einer schonenden Reinigung) und in einer definierten horizontalen 2 und vertikalen 3 Bewegung, die einen Wischzyklus 4 ergeben.

### Beispiele

Die Erfindung wird in den nachfolgenden Beispielen näher erläutert.

### Beispiel 1: Herstellen eines Vertikalvliesstoffes

Es wird ein Vertikalvliesstoff in Anlehnung an das in der CN 104805597 beschriebene Verfahren (Anyou-Verfahren) hergestellt. Hierzu wird über eine Krempel ein Flor gebildet. Die Fasermischung besteht aus 70 Gew.% Polyesterfasern (Grisuten^{®}, Märkische Faser GmbH) mit einem Schmelzpunkt von 250 °C und 30 Gew.% gekräuselte Binderfasern (EMF, Huvis) mit einem Mantelschmelzpunkt von 148 °C. Der Flor wird mit Hilfe einer oszillierenden Legeeinheit ziehharmonikaartig mit einer Dicke von 50 mm abgelegt und anschließend mit Hilfe eines Doppelbandofens thermisch verfestigt. Der so gebildete Vertikalvliesstoff wird daraufhin auf eine Dicke von 5 mm mit Hilfe einer Spaltmaschine (Fecken-Kirfel) gespalten.

### Beispiel 2: Bestimmung der Reinigungsleistung des Vertikalvliesstoffs in einem ex-vivo Wundmodell

Der in Beispiel 1 hergestellte Vertikalvliesstoff wurde durch Stanzen auf eine Größe von 10 cm x 10 cm konfektioniert. Die Reinigungsleistung des Vertikalvliesstoffs wurde mit Hilfe eines ex-vivo Wundmodels auf Schweinehaut bewertet. Die zu reinigende Wunde wurde wie folgt vorbereitet: Zunächst wurde die Schweinehaut mit der Hautinnenseite nach oben in einem Rahmen (Fläche für Wunde 10 cm x 10 cm) fixiert, mit einem Skalpell definiert in Form eines Gittermusters eingeritzt. Anschließend wurde die Haut mit einem Flammbierbrenner leicht verbrannt (20 Sekunden, mittlere Flamme, Abstand ca. 20 cm), so dass durch Aufklaffen der Schnitte ein unebener Wundgrund entsteht. Austretendes Fett wurde vorsichtig mit einem Papiertuch durch Tupfen entfernt. Anschließend wurden 4 mL Eiweißlösung (100 g/L Eiweißpulver (bspw. Body&Fit Egg White Powder in Phosphat gepufferter Salzlösung (8,0 g/L Natriumchlorid (NaCl), 0,2 g/L Kaliumchlorid (KCI), 1,42 g/L Dinatriumhydrogenphosphat (Na2HPO4) oder 1,78 g/L Dinatriumhydrogenphosphat Dihydrat (Na2HPO4• 2 H2O), 0,27 g/L Kaliumdihydrogenphosphat (KH2PO4)) gelöst) aufgetragen und erneut mit Hilfe eines Flammbierbrenners eingebrannt (30 Sekunden, mittlere Flamme, Abstand ca. 20 cm). Die Eiweißlösung eignet sich zur Simulierung von Fibrinbelägen in der Wunde. In einem letzten Schritt werden 2 g künstliche Exsudatlösung (5 % Blanose, 40 g/L Eiweißpulver, 1 g/L Zucker, Kunstblut (150 Tropfen auf 300 mL, 2g/L Sonnenblumen Öl, 3,7 g/L Natrium Carbonat, 3 g/L Quarzsand alles gelöst in PBS) mittels eines Holzspachtels auf die Wunde aufgetragen und erneut eingebrannt, bis sich (teilweise) eine schwarze Kruste bildet (30 Sekunden, mittlere Flamme, Abstand ca. 20 cm). Die simulierte Wunde wird 2 Stunden bei Raumtemperatur ruhen gelassen, damit zuvor appliziertes Exsudat nicht leicht wieder entfernt werden kann.

Zur Reinigung wurde der Vertikalvliesstoff auf einer Seite (10 cm x 10 cm) mit 5 Sprühstößen mit Wasser angefeuchtet und mit der angefeuchteten Seite zur Wundoberfläche zeigend, eine Minute auf die Wunde gelegt. Anschließend erfolgte die Reinigung der Wundfläche mit geringem Druck (zur Simulation einer schonenden Reinigung) und in einer definierten horizontalen und vertikalen Bewegung, die einen Wischzyklus ergeben (siehe Figur 5). Die Reinigung erfolgt für 3 Minuten mit 11 Wischzyklen.

Die Auswertung der Reinigungsleistung erfolgte über Bildanalyse der verbrannten (schwarzen) Bereiche vor und nach der Wundreinigung. Die präparierten Schweinehäute wurden mit einer Spiegelreflexkamera (Canon D70) mit Festbrennweitenobjektiv (60 mm) mit Hilfe eines Reprostativs aufgenommen. Dabei wurde darauf geachtet, dass die Beleuchtung und der Abstand (resultierende Vergrößerung) zwischen Objekt und Objektiv über alle Aufnahmen gleich waren. Im Zuge der Untersuchungen wurde ein Metallrahmen zur Glättung der Schweinehäute aufgelegt. Über eine Schwellwertsetzung und einen 15 x 15 Pixel Medianfilter wurden die schwarzen Bereiche im Bild ausfindig gemacht und grün markiert. Für alle Bilder wurde eine Obergrenze von 80 im Histogramm gewählt. Dies, und das gleichbleibende Aufnahmesetup stellen sicher, dass die Ergebnisse aller Proben vergleichbar sind. Als letzter Schritt wurde der sinnvoll auszuwertende Bereich des Bildes begrenzt (ROI). Aufgrund der Kalibrierung der Bilder bei der Aufnahme war es anschließend möglich die markierten Bereiche als Flächenanteile in µm anzugeben.

**Tabelle 1: Vergleich der Reinigungsleistung von Baumwollgaze, Debrisoft^{®} und eines Vertikalvliesstoffs**

| | Fläche vor Reinigung [mm²] | Fläche nach Reinigung [mm²] | Reinigungsleistung in [%] |
|---|---|---|---|
| Baumwollgaze | 629,0 | 286,0 | 54,5 |
| Baumwollgaze | 1491,0 | 1010,0 | 32,3 |
| Debrisoft^{®} | 3162,0 | 1276,0 | 59,6 |
| Debrisoft^{®} | 3374,0 | 1289,0 | 61,7 |
| Vertikalvliesstoff | 2993,0 | 189,0 | 93,7 |
| Vertikalvliesstoff | 2904,0 | 353,0 | 87,8 |

### Beispiel 3 Bestimmung der Aufnahme von Wundbestandteilen durch einen Vertikalvliesstoff

Zur Bestimmung der Aufnahme von Wundbestandteilen im Vertikalvliesstoff wurden Rasterelektronen-Mikroskopieaufnahmen angefertigt (Vgl. Figur 2). Wie man sieht werden Wundbestandteile in Porenstruktur des Vertikalvliesstoffs aufgenommen (Figur 2 Bereich innerhalb gepunktetem Oval). Dahingegen erfolgt die Absorption bei Baumwollgaze (Figur 3 Bereich innerhalb Strich-Punkt Oval) und Debrisoft (Figur 4 Bereich innerhalb Oval) nur auf der Oberfläche, bzw. im oberen Teil der Struktur.

## Patentansprüche

1. Steriler Artikel zur mechanischen Wundreinigung, umfassend einen Vertikalvliesstoff mit einer Dicke von 1,5 mm bis 50 mm, wobei der Vertikalvliesstoff Bindefasern enthält, **dadurch gekennzeichnet, dass** mindestens eine Seite des Vertikalvliesstoffs für den direkten Kontakt mit der Wunde vorgesehen ist, wobei der Vertikalvliesstoff vertikal zur Richtung seiner Dicke gefaltet ist.

2. Steriler Artikel nach Anspruch 1, **dadurch gekennzeichnet, dass** der Vertikalvliesstoff thermisch verfestigt ist.

3. Steriler Artikel nach Anspruch 1 oder 2, **dadurch gekennzeichnet, dass** mindestens eine, vorzugsweise beide Seiten des Vertikalvliesstoffs keine mit dem Vertikalvliesstoff unlösbar verbundene Schichten aufweisen.

4. Steriler Artikel nach einem oder mehreren der vorangehenden Ansprüche, **dadurch gekennzeichnet, dass** der Vertikalvliesstoff Matrixfasern enthält, die Polyester, Polyamide, Polyolefine, Polyacrylnitril, und/oder Polyvinylalkohole enthalten.

5. Steriler Artikel nach einem oder mehreren der vorangehenden Ansprüche, **dadurch gekennzeichnet, dass** der Anteil der Matrixfasern im Vertikalvliesstoff vorzugsweise mindestens 20 Gew.%, beispielsweise von 20 bis 100 Gew.%, noch bevorzugter mindestens 25 Gew. %, beispielsweise von 25 bis 80 Gew.%, noch bevorzugter mindestens 30 Gew.%, beispielsweise von 30 bis 70 Gew.% beträgt, jeweils bezogen auf das Gesamtgewicht des Vertikalvliesstoffs.

6. Steriler Artikel nach einem oder mehreren der vorangehenden Ansprüche, **dadurch gekennzeichnet, dass** der Vertikalvliesstoff Bikomponentenfasern, insbesondere in einem Anteil von mindestens 30 Gew.%, jeweils bezogen auf das Gesamtgewicht des Vertikalvliesstoffs aufweist.

7. Steriler Artikel nach einem oder mehreren der vorangehenden Ansprüche, **dadurch gekennzeichnet, dass** der Vertikalvliesstoff gekräuselte Fasern, vorzugsweise in einem Anteil von mindestens 30 Gew.%, jeweils bezogen auf das Gesamtgewicht des Vertikalvliesstoffs aufweist.

8. Steriler Artikel nach einem oder mehreren der vorangehenden Ansprüche, **dadurch gekennzeichnet, dass** der Vertikalvliesstoff gekräuselte Bindefasern aufweist, vorzugsweise in einem Anteil von mindestens 20 Gew.%, jeweils bezogen auf das Gesamtgewicht des Vertikalvliesstoffs.

9. Steriler Artikel nach einem oder mehreren der vorangehenden Ansprüche, **dadurch gekennzeichnet, dass** der Vertikalvliesstoff Stapelfasern enthält, vorzugsweise mit einer Stapellänge zwischen 20 und 150 mm.

10. Steriler Artikel nach einem oder mehreren der vorangehenden Ansprüche, **dadurch gekennzeichnet, dass** der Vertikalvliesstoff Fasern mit einem Fasertiter im Bereich von 0,9 bis 100 dtex (g/10.000 m), noch bevorzugter zwischen 1,5 und 30 dtex, insbesondere zwischen 3 und 11 dtex aufweist.

11. Steriler Artikel nach einem oder mehreren der vorangehenden Ansprüche, **dadurch gekennzeichnet, dass** der sterile Artikel der sterile Vertikalvliesstoff ist; oder dass der sterile Artikel der Vertikalvliesstoff ist, der steril verpackt ist.

12. Verwendung eines sterilen Artikels gemäß mindestens einem der Ansprüche 1 bis 11 als Reinigungsmittel, insbesondere für eine menschliche oder tierische Körperoberfläche.

## Claims

1. Sterile article for mechanical wound cleaning, comprising a vertically-lapped nonwoven having a thickness of 1.5 mm to 50 mm, wherein the vertically-lapped nonwoven contains binding fibres, **characterized in that** at least one side of the vertically-lapped nonwoven is intended for direct contact with the wound, wherein the vertically-lapped nonwoven is folded vertically in the direction of its thickness.

2. Sterile article according to Claim 1, **characterized in that** the vertically-lapped nonwoven is thermally consolidated.

3. Sterile article according to Claim 1 or 2, **characterized in that** at least one, preferably both sides of the vertically-lapped nonwoven have no layers inseparably connected to the vertically-lapped nonwoven.

4. Sterile article according to one or more of the preceding claims, **characterized in that** the vertically-lapped nonwoven contains matrix fibres which contain polyesters, polyamides, polyolefins, polyacrylonitrile, and/or polyvinyl alcohols.

5. Sterile article according to one or more of the preceding claims, **characterized in that** the fraction of the matrix fibres in the vertically-lapped nonwoven is preferably at least 20% by weight, for example from 20% to 100% by weight, more preferably at least 25% by weight, for example from 25% to 80% by weight, more preferably at least 30% by weight, for example from 30% to 70% by weight, based in each case on the total weight of the vertically-lapped nonwoven.

6. Sterile article according to one or more of the preceding claims, **characterized in that** the vertically-lapped nonwoven has bicomponent fibres, in particular in a fraction of at least 30% by weight, based in each case on the total weight of the vertically-lapped nonwoven.

7. Sterile article according to one or more of the preceding claims, **characterized in that** the vertically-lapped nonwoven has crimped fibres, preferably in a fraction of at least 30% by weight, based in each case on the total weight of the vertically-lapped nonwoven.

8. Sterile article according to one or more of the preceding claims, **characterized in that** the vertically-lapped nonwoven has crimped binding fibres, preferably in a fraction of at least 20% by weight, based in each case on the total weight of the vertically-lapped nonwoven.

9. Sterile article according to one or more of the preceding claims, **characterized in that** the vertically-lapped nonwoven contains staple fibres, preferably having a staple length of between 20 and 150 mm.

10. Sterile article according to one or more of the preceding claims, **characterized in that** the vertically-lapped nonwoven has fibres having a fibre linear density in the range from 0.9 to 100 dtex (g/10 000 m), more preferably between 1.5 and 30 dtex, more particularly between 3 and 11 dtex.

11. Sterile article according to one or more of the preceding claims, **characterized in that** the sterile article is the sterile vertically-lapped nonwoven or **in that** the sterile article is the vertically-lapped nonwoven which is sterile-packaged.

12. Use of a sterile article according to at least one of Claims 1 to 11 as a cleansing product, in particular for a human or animal body surface.

## Revendications

1. Article stérile pour le nettoyage mécanique de plaies, comprenant un non-tissé vertical d'une épaisseur de 1,5mm à 50mm, le non-tissé vertical contenant des fibres de liaison, **caractérisé en ce qu'**au moins un côté du non-tissé vertical est prévu pour un contact direct avec la plaie, le non-tissé vertical étant plié verticalement dans la direction de son épaisseur.

2. Article stérile selon la revendication 1, **caractérisé en ce que** le non-tissé vertical est thermolié.

3. Article stérile selon la revendication 1 ou la revendication 2, **caractérisé en ce qu'**au moins un côté, de préférence les deux côté, du non-tissé vertical ne présente pas de couches liées de manière indissociable au non-tissé vertical.

4. Article stérile selon une ou plusieurs des revendications précédentes, **caractérisé en ce que** le non-tissé vertical contient des fibres de matrice qui contiennent du polyester, des polyamides, des polyoléfines, du polyacrylonitrile, et/ou des alcools polyvinyliques.

5. Article stérile selon une ou plusieurs des revendications précédentes, **caractérisé en ce que** la proportion de fibres de matrice dans le non-tissé vertical est de préférence d'au moins 20% en poids, par exemple de 20 à 100% en poids, de façon encore préférée d'au moins 25% en poids, par exemple de 25 à 80% en poids, de façon encore préférée d'au moins 30% en poids, par exemple de 30 à 70% en poids, dans chaque cas par rapport au poids total du non-tissé vertical.

6. Article stérile selon l'une ou plusieurs des revendications précédentes, **caractérisé en ce que** le non-tissé vertical présente des fibres bicomposantes, en particulier dans une proportion d'au moins 30% en poids, dans tous les cas par rapport au poids total du non-tissé vertical.

7. Article stérile selon une ou plusieurs des revendications précédentes, **caractérisé en ce que** le non-tissé vertical présente des fibres frisées, de préférence dans une proportion d'au moins 30% en poids, dans tous les cas par rapport au poids total du non-tissé vertical.

8. Article stérile selon une ou plusieurs des revendications précédentes, **caractérisé en ce que** le non-tissé vertical présente des fibres de liaison frisées, de préférence dans une proportion d'au moins 20% en poids, dans tous les cas par rapport au poids total du non-tissé vertical.

9. Article stérile selon une ou plusieurs des revendications précédentes, **caractérisé en ce que** le non-tissé vertical contient des fibres coupées, de préférence avec une longueur de coupe comprise entre 20 et 150mm.

10. Article stérile selon une ou plusieurs des revendications précédentes, **caractérisé en ce que** le non-tissé vertical comprend des fibres ayant un titre de fibres compris entre 0,9 et 100dtex (g/10000m), de préférence encore entre 1,5 et 30dtex, en particulier entre 3 et 11dtex.

11. Article stérile selon une ou plusieurs des revendications précédentes, **caractérisé en ce que** l'article stérile est le non-tissé vertical stérile ; ou **en ce que** l'article stérile est le non-tissé vertical qui est emballé de manière stérile.

12. Utilisation d'un article stérile selon au moins l'une des revendications 1 à 11 comme moyen de nettoyage, notamment d'une surface corporelle humaine ou animale.
